# EUROPÄISCHE PATENTSCHRIFT

(11) **EP 2 096 984 B1**
(45) Veröffentlichungstag und Bekanntmachung des Hinweises auf die Patenterteilung: **04.01.2012**
(21) Anmeldenummer: 06776702.0
(22) Anmeldetag: 09.08.2006
(51) Int. Cl.: A61B 5/00

(54) **MEDIZINISCHE MESSVORRICHTUNG**
MEDICAL MEASURING DEVICE
DISPOSITIF DE MESURE MEDICAL

(30) Priorität: 09.08.2005 DE 102005038035; 25.10.2005 DE 102005051030
(43) Veröffentlichungstag der Anmeldung: 09.09.2009
(73) Patentinhaber: Flore, Ingo, 44141 Dortmund (DE)
(72) Erfinder: Cho, Ok Kyung, 58239 Schwerte (DE); Kim, Yoon Ok, 58239 Schwerte (DE)
(74) Vertreter: Isfort, Olaf
(86) Internationale Anmeldenummer: PCT/EP2006/007874
(87) Internationale Veröffentlichungsnummer: WO 2007/017263

(56) Entgegenhaltungen:
- EP-A- 1 491 134
- WO-A1-99/62399
- US-A- 5 237 178
- US-A1- 2004 034 293
- US-B1- 6 790 178
- LEPRETRE PIERRE-MARIE ET AL: "Effect of exercise intensity on relationship between VO2max and cardiac output." MEDICINE AND SCIENCE IN SPORTS AND EXERCISE AUG 2004, Bd. 36, Nr. 8, August 2004 (2004-08), Seiten 1357-1363, XP002428499 ISSN: 0195-9131

## Beschreibung

Die Erfindung betrifft eine Messvorrichtung zur nicht-invasiven Bestimmung von physiologischen Parametern, mit wenigstens einer optischen Messeinheit zur Erzeugung von oximetrischen und/oder plethysmographischen Messsignalen, und mit einer Auswertungseinheit zur Verarbeitung der Messsignale.

Die Versorgung des Körpergewebes mit Sauerstoff gehört bekanntlich zu den wichtigsten Vitalfunktionen des Menschen. Aus diesem Grund sind oximetrische Diagnosemodalitäten heutzutage von großer Bedeutung in der Medizin. Routinemäßig werden sogenannte Pulsoximeter eingesetzt. Derartige Pulsoximeter umfassen typischerweise zwei Lichtquellen, die rotes bzw. infrarotes Licht unterschiedlicher Wellenlänge in das Körpergewebe einstrahlen. Das Licht wird im Körpergewebe gestreut und teilweise absorbiert. Das gestreute Licht wird schließlich mittels eines Lichtsensors in Form einer geeigneten Photozelle detektiert. Typischerweise verwenden kommerzielle Pulsoximeter zum einen Licht im Wellenlängenbereich von 660 nm. In diesem Bereich ist die Lichtabsorption von Oxihämoglobin und Desoxihämoglobin stark unterschiedlich. Dementsprechend variiert die Intensität des mittels des Photosensors detektierten, gestreuten Lichts in Abhängigkeit davon, wie stark das untersuchte Körpergewebe von sauerstoffreichem, bzw. sauerstoffarmem Blut durchblutet ist. Zum anderen wird üblicherweise Licht im Wellenlängenbereich von 810 nm verwendet. Diese Lichtwellenlänge liegt im sogenannten nahen infraroten Spektralbereich. Die Lichtabsorption von Oxihämoglobin und Desoxihämoglobin ist in diesem Spektralbereich im Wesentlichen gleich. Die bekannten Pulsoximeter sind außerdem in der Lage, ein plethysmographisches Signal, d. h. ein Volumenpulssignal zu erzeugen, das die während des Herzschlags veränderliche Blutmenge in dem von dem Pulsoximeter erfassten Mikrogefäßsystem wiedergibt (sog. Photoplethysmographie). Bei Verwendung unterschiedlicher Lichtwellenlängen in den oben erwähnten Spektralbereichen kann aus der unterschiedlichen Lichtabsorption auf den Sauerstoffgehalt des Blutes (Sauerstoffsättigung) zurückgeschlossen werden. Die üblichen Pulsoximeter werden entweder an der Fingerspitze eines Patienten oder auch am Ohrläppchen eingesetzt. Es wird dann das Volumenpulssignal aus der Blutperfusion des Mikrogefäßsystems in diesen Bereichen des Körpergewebes erzeugt.

Aus der WO 00/69328 A1 ist ein besonders flexibel einsetzbares oximetrisches Diagnosegerät bekannt. Dieses vorbekannte Gerät ist handführbar ausgebildet, so dass es an beliebigen Messorten am menschlichen Körper eingesetzt werden kann. Das vorbekannte Gerät erlaubt gleichsam ein systematisches Abtasten ("Scannen") des Körpers eines Patienten. Eine Fixierung des Diagnosegerätes - wie bei üblichen Pulsoximetern - kann bei dem aus der genannten Druckschrift bekannten Gerät entfallen.

Die genannte WO 00/69328 A1 spricht außerdem die Einsetzbarkeit des oximetrischen Diagnosegerätes zur ortsaufgelösten Erkennung von Entzündungen, Tumoren und Arterioskleroseerkrankungen im hautoberflächennahen Körpergewebe eines Patienten an. Derartige Erkrankungen bewirken eine Veränderung der Durchblutung des Körpergewebes. Durch die ortsaufgelöste oximetrische Abtastung des Körpers lassen sich mit dem vorbekannten Gerät Veränderungen der Durchblutung, die auf eine entsprechende Erkrankung hindeuten, erkennen und lokalisieren.

Ein Oximeter gemäß der Präambel von Anspruch 1 ist aus der WO 99/62399 bekannt.

Das EKG (Elektrokardiogramm) dürfte die am meisten eingesetzte Untersuchungsmodalität zur Diagnose von Herz-Kreislauf-Erkrankungen sein. Mittels eines EKG-Gerätes werden mit zwei oder mehr EKG-Elektroden elektrische Signale von dem Körper des zu untersuchenden Patienten abgeleitet. Das so gewonnene EKG gibt die bioelektrischen Spannungen, die bei der Erregungsausbreitung und -rückbildung am Herzen entstehen, wieder. Das EKG enthält zahlreiche diagnostisch auswertbare Parameter. Zum Zeitpunkt der Kontraktion des Herzmuskels während eines Herzschlags zeigt das EKG eine deutliche Spitze, die auch als R-Zacke bezeichnet wird. Weiterhin enthält das EKG die der R-Zacke vorangehende, so genannte P-Welle. Der R-Zacke folgt wiederum die so genannte T-Welle. Die Minima im EKG unmittelbar vor und unmittelbar nach der R-Zacke werden mit Q bzw. S bezeichnet. Für die Herz-Kreislauf-Diagnostik interessante Parameter sind die Dauer oder P-Welle sowie die Amplitude der P-Welle, die Dauer des PQ-Intervalls, die Dauer des QRS-Komplexes, die Dauer des QT-Intervalls sowie die Amplitude der T-Welle. Sowohl aus den Absolutwerten der genannten Parameter wie auch aus den Verhältnissen der Parameter kann auf den Gesundheitszustand des Herz-Kreislauf-Systems geschlossen werden. Vorrichtungen und Verfahren zur EKG-Messung sind beispielsweise aus den Druckschriften US 6,331,162 oder US 4,960,126 vorbekannt.

Zur Bestimmung von weiteren physiologischen Parametern, wie z. B. Körperfettgehalt, ist das Prinzip der bioelektrischen Impedanzmessung beispielsweise aus der US 6,714,814 bekannt. Die Zusammensetzung des Körpergewebes kann aber auch optisch bestimmt werden. Das Prinzip der optischen Bestimmung des Körperfettgehalts mittels Infrarotlicht ist beispielsweise in der US 4,928,014 beschrieben.

Der vorliegenden Erfindung liegt die Aufgabe zugrunde, eine Vorrichtung zur nicht-invasiven Bestimmung von physiologischen Parametern bereit zu stellen, die gegenüber dem Stand der Technik verbessert und hinsichtlich ihrer Funktionalität erweitert ist. Insbesondere soll ein Gerät geschaffen werden, das eine möglichst zuverlässige Erkennung und Lokalisierung von Erkrankungen, wie Entzündungen, Tumor- bzw. Krebserkrankungen (Hautkrebs, Melanome) sowie Gefäßerkrankungen, ermöglicht. Außerdem soll optional die Möglichkeit bestehen, mittels des Gerätes die (kardiovaskuläre) Fitness des Benutzers einzuschätzen. Dabei soll das Gerät auch zur Selbstdiagnose genutzt werden können.

Diese Aufgabe löst die Erfindung ausgehend von einer Messvorrichtung gemäß Anspruch 1.

Kerngedanke der Erfindung ist es, die mittels der optischen Messeinheit gewonnenen oximetrischen und/oder plethysmographischen Messsignale heranzuziehen, um nicht nur - wie z. B. bei den bekannten oximetrischen Diagnosegeräten - die lokale Sauerstoffkonzentration am jeweiligen Messort, sondern insbesondere auch den lokalen Sauerstoffverbrauch als wichtigen Indikator für die lokale metabolische Aktivität zu bestimmen. Erkrankungen können mit der erfindungsgemäßen Messvorrichtung anhand von pathologischen Veränderungen des Metabolismus erkannt und lokalisiert werden.

Gemäß einer bevorzugten Ausgestaltung weist die erfindungsgemäße Messvorrichtung zusätzlich eine Einheit zur Erfassung von lokalen Gewebeparametern, wie Fettgehalt, Wassergehalt und/oder Durchblutung auf, wobei die Auswertungseinheit in diesem Fall eingerichtet ist zur Bestimmung des wenigstens einen lokalischen metabolischen Parameters aus den Signalen der optischen Messeinheit und den Gewebeparametern.

Ein wichtiger lokaler Gewebeparameter im Sinne der Erfindung ist beispielsweise die Durchblutung. Damit sind die durchblutungsbedingten Volumenschwankungen des untersuchten Körpergewebes gemeint. Zur Erfassung der Durchblutung kann die erfindungsgemäße Messvorrichtung insofern mit einer Plethysmographieeinheit herkömmlicher Art (z. B. Photoplethysmograph) ausgestattet sein. Somit kann die optische Messeinheit der erfindungsgemäßen Messvorrichtung gleichzeitig zur Erfassung der lokalen Gewebeparameter genutzt werden.

Die Erfindung basiert u. a. auf der Erkenntnis, dass durch die Kombination der Erfassung von oximetrischen und plethysmographischen Signalen die Möglichkeit eröffnet wird, lokale metabolische Parameter zu bestimmen.

Für die Ermittlung des lokalen Sauerstoffverbrauchs sollte mittels der erfindungsgemäßen Messvorrichtung zusätzlich zur oximetrisch bestimmten arteriellen Sauerstoffkonzentration auch die kapillare Sauerstoffkonzentration im Gewebe bestimmt werden können. Hierzu muss allerdings die Zusammensetzung des untersuchten Körpergewebes bekannt sein. Entscheidende Parameter sind der lokale Fettgehalt und/oder der Wassergehalt des Körpergewebes. Diese Parameter können beispielsweise mittels bioelektrischer Impedanzmessung erfasst werden. Gemäß einer sinnvollen Ausgestaltung der Erfindung ist eine herkömmliche (optische) Oximetrieeinheit mit einer bioelektrischen Impedanzmesseinheit in einem einzigen Gerät kombiniert. Aus den mittels der bioelektrischen Impedanzmesseinheit gewonnenen Messsignalen kann die Zusammensetzung des untersuchten Körpergewebes bestimmt werden. Auf dieser Grundlage kann dann aus den oximetrischen Signalen mittels der Auswertungseinheit der Messvorrichtung die kapillare Sauerstoffsättigung im Gewebe ermittelt werden.

Eine sinnvolle Weiterbildung der erfindungsgemäßen Messvorrichtung sieht vor, dass die bioelektrische Impedanzmesseinheit außerdem zur Erfassung von globalen Gewebeparametern, wie globaler Fettgehalt und/oder globaler Wassergehalt, ausgebildet ist. Hierdurch wird die Funktionalität der erfindungsgemäßen Messvorrichtung erweitert. Die bioelektrische Impedanzmesseinheit der erfindungsgemäßen Messvorrichtung kann derart ausgestattet sein, dass damit sowohl lokale wie auch globale Gewebeparameter gemessen werden können.

Die Zusammensetzung des Körpergewebes kann mit der erfindungsgemäßen Messvorrichtung auch optisch bestimmt werden. Hierzu kann die Einheit zur Erfassung von lokalen Gewebeparametern eine optische Strahlungsquelle und einen Photosensor umfassen. Das Prinzip der optischen Bestimmung des Körperfettgehalts mittels Infrarotlicht ist aus dem Stand der Technik bekannt.

Gemäß einer vorteilhaften Ausgestaltung umfasst die erfindungsgemäße Vorrichtung einen Wärmesensor zur Bestimmung der über die Hautoberfläche abgegebenen Wärme, wobei die Auswertungseinheit zur Bestimmung der lokalen metabolischen Parameter unter Berücksichtigung der Signale des Wärmesensors eingerichtet ist. Vorzugsweise ist mittels des Wärmesensors eine orts-, zeit- und tiefenaufgelöste Wärmemessung am Messort möglich. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No.9, Seiten 2953 bis 2956). Insgesamt liefert der Wärmesensor Daten, die mit Vorteil zur Bestimmung von metabolischen Parametern im Sinne der Erfindung genutzt werden können.

Die arterielle Sauerstoffsättigung (SaO₂) und die venöse Sauerstoffsättigung (SvO₂) bestimmen abhängig von der Art des untersuchten Gewebes die kapillare (arteriovenöse) Sauerstoffsättigung (StO₂). Es gilt:

K * SvO₂ + (1 - K) * SaO₂ = StO₂,

wobei K ein gewebeabhängiger Korrekturfaktor ist, der vom Volumenverhältnis von Arterien zu Venen im untersuchten Gewebe abhängt. Im Mittel liegt dieser Wert etwas unter 0,5. Der für das jeweilige Gewebe maßgebliche Wert kann gemäß der Erfindung durch bioelektrische Impedanzmessung ermittelt werden, um dann aus der obigen Formel die venöse Sauerstoffsättigung zu bestimmen. Mittels Wärmemessung und/oder bioelektrischer Impedanz (Impedanzplethysmographie) kann die Durchblutung V, d. h. die durchblutungsbedingte Volumenschwankung des Gewebes bestimmt werden. Nach der Beziehung

VO₂ = V * (SaO₂ - SvO₂)

kann dann schließlich der lokale Sauerstoffverbrauch VO₂ berechnet werden, der ein Maß für die metabolische Aktivität am Messort darstellt.

Des Weiteren kann die erfindungsgemäße Messvorrichtung einen optischen Sensor zur ortsaufgelösten Bestimmung des Hautkolorits umfassen. Auch anhand von lokalen Verfärbungen der Haut können Erkrankungen, wie zum Beispiel Entzündungen, Melanome usw., detektiert werden.

Durch eine zusätzliche EKG-Einheit zur Erfassung eines EKG-Signals über zwei oder mehr EKG-Elektroden wird der Funktionsumfang der erfindungsgemäßen Messvorrichtung vorteilhaft erweitert. Gemäß der Erfindung werden mittels der Messvorrichtung plethysmographische Signale und EKG-Signale kombiniert erfasst und ausgewertet. Die Auswertungseinheit der Messvorrichtung kann dann mit Vorteil zur Auswertung des zeitlichen Verlaufs der Volumenpulssignale und der EKG-Signale eingerichtet sein. Mittels einer geeigneten Programmsteuerung ist die Auswertungseinheit der erfindungsgemäßen Messvorrichtung dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund ihrer Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Messvorrichtung erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden. Es können in diese Auswertung auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar.

Durch die Kombination der Auswertung des EKG-Signals und des Volumenpulssignals bei der automatischen Auswertung ist die erfindungsgemäße Messvorrichtung zur funktionalen Bewertung des Gefäßsystems des Patienten selbsttätig in der Lage. Auf der Grundlage der automatisch ausgewerteten Signale kann die erfindungsgemäße Vorrichtung den (globalen) kardiovaskulären Zustand oder allgemein die Fitness des Benutzers grob einschätzen und bei Anzeichen einer Arteriosklerose oder sonstiger Herz-Kreislauf-Probleme ein entsprechendes Warnsignal oder einen leicht interpretierbaren Fitness- oder Risikoindikator für den Benutzer der Vorrichtung erzeugen. Somit kann die erfindungsgemäße Messvorrichtung vorteilhaft zur Selbstdiagnose von Herz-Kreislauf-Erkrankungen verwendet werden.

Besonders vorteilhaft ist die erfindungsgemäße Kombination der vorgenannten Messverfahren, nämlich der Oximetrie, der bioelektrischen Impedanzmessung und der Wärmemessung. Mittels der Auswertungseinheit der Vorrichtung können sämtliche Messsignale ausgewertet werden, um daraus die arterielle, die kapillare und die venöse Sauerstoffsättigung und daraus wiederum die lokale Stoffwechselaktivität zu bestimmen. Dadurch wird eine hohe Effektivität und Zuverlässigkeit bei der Erkennung und Lokalisierung von pathologischen Veränderungen erreicht. Diese wird durch zusätzliche Berücksichtigung des lokalen Hautkolorits noch verbessert. Die zusätzliche EKG-Messung erlaubt, wie oben ausgeführt, Aussagen bezüglich des Status des Herz-Kreislauf-Systems des Benutzers. Sämtliche Parameter können mit Vorteil zu einem globalen Index zusammengefasst werden, der für den Benutzer leicht interpretierbar ist und ihm einen direkten und fundierten Hinweis auf seinen allgemeinen Gesundheitszustand gibt.

Die erfindungsgemäße Vorrichtung kann weiterhin eine Datenübertragungsschnittstelle umfassen zur Übertragung der mittels der Auswertungseinheit ermittelten Parameter an einen Personalcomputer (des Arztes), beispielsweise über das Internet, oder an ein anderes Gerät. Hierbei kann es sich um eine übliche drahtgebundene oder auch um eine drahtlose Schnittstelle (beispielsweise nach dem DECT-, GSM-, UMTS- oder Bluetooth-Standard) handeln. Eine Datenübertragung über Infrarotdatenkommunikation oder Ultraschall ist ebenfalls denkbar.

Eine besonders sinnvolle Ausgestaltung der erfindungsgemäßen Messvorrichtung ergibt sich, wenn diese eine Speichereinheit zur Speicherung der mittels der Auswertungseinheit ermittelten Parameter aufweist. Mittels der Speichereinheit können einerseits der Verlauf einer Erkrankung und andererseits die Effekte einer entsprechenden Therapie verfolgt und dokumentiert werden. Andererseits können die in der Speichereinheit abgespeicherten Daten vom behandelnden Arzt ausgelesen und ausgewertet werden, um eine detaillierte Zustandsdiagnostik durch den Arzt zu ermöglichen. Sinnvoll ist es weiterhin, wenn die erfindungsgemäße Vorrichtung eine Diagnoseeinheit zur Bewertung der mittels der Auswertungseinheit ermittelten Parameter und zur Registrierung von Veränderungen der Parameter in Abhängigkeit vom Messort und von der Messzeit aufweist. Demgemäß hat die erfindungsgemäße Vorrichtung einen modularen Aufbau. Die Auswertungseinheit ist lediglich dafür zuständig, die erfassten Signale auszuwerten, um daraus die für die Diagnostik erforderlichen Parameter in der oben beschriebenen Art und Weise zu bestimmen. Diese Parameter werden dann von der Diagnoseeinheit weiter verarbeitet, um daraus Rückschlüsse bezüglich etwaiger Erkrankungen zu ziehen. Die Diagnoseeinheit ist auch dafür zuständig, insbesondere bei Verwendung der Messvorrichtung zur Selbstdiagnose durch einen Benutzer, das Vorliegen einer Erkrankung automatisch zu erkennen und gegebenenfalls ein entsprechendes Warnsignal für den Benutzer zu erzeugen.

Sinnvollerweise ist also die Diagnoseeinheit der erfindungsgemäßen Messvorrichtung zur Bestimmung des Status des Herz-Kreislauf-Systems aus den mittels der Auswertungseinheit ermittelten Parametern eingerichtet. Gemäß einer besonders vorteilhaften Ausgestaltung der Erfindung ist die Diagnoseeinheit außerdem zur Berechnung eines globalen Fitnessindex auf der Basis des Status des Herz-Kreislauf-Systems und den (mittels bioelektrischer Impedanzmessung erfassten) globalen Gewebeparametern eingerichtet. Somit können die globalen Gewebeparameter genutzt werden, um den globalen Fitnessindex zu erhalten, der besonders aufschlussreich Auskunft über den momentanen Gesundheitszustand des Benutzers gibt. Zur Bestimmung des globalen Fitnessindex können sämtliche erfassten Messwerte des Benutzers einbezogen werden. Gegebenenfalls wird eine Mittelung über einen vorgebbaren Zeitraum durchgeführt. Neben den kardiovaskulären Messwerten und den globalen Gewebeparametern (globaler Fettgehalt, globaler Wassergehalt) können auch die lokalen Gewebeparameter sowie die lokalen metabolischen Parameter (z. B. lokaler Sauerstoffverbrauch) mit berücksichtigt werden. Das Ergebnis ist dann der globale Fitnessindex als einzelner Wert, der für den Benutzer der Messvorrichtung besonders einfach interpretierbar ist.

Zumindest die optische Messeinheit der erfindungsgemäßen Messvorrichtung arbeitet auf der Basis von optischen Messverfahren. Als Strahlungsquelle kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens drei unterschiedlichen Lichtwellenlängen gemessen wird, um daraus die Sauerstoffkonzentration des Blutes und die Durchblutung des Gewebes zu bestimmen.

Gemäß einer sinnvollen Ausgestaltung weist die optische Messeinheit der erfindungsgemäßen Messvorrichtung wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist.

Erfindungsgemäß weist die Messvorrichtung wenigstens zwei Strahlungsquellen auf, welche unterschiedliche Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Messvorrichtung vorteilhafterweise auch eine nicht-invasive Bestimmung des Blutglukosespiegels.

Die zwei Strahlungsquellen der optischen Messeinheit der erfindungsgemäßen Messvorrichtung sind so ausgelegt sein, dass die von diesen jeweils bestrahlten Volumenbereiche hinsichtlich der Durchblutung mit sauerstoffarmem bzw. sauerstoffreichem Blut unterschiedlich betroffen sind. Dies wird dadurch erreicht, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Die Auswertungseinheit der erfindungsgemäßen Messvorrichtung kann mit Vorteil zur Bestimmung des wenigstens einen lokalen metabolischen Parameters aus der von dem Körpergewebe gestreuten und/oder transmittierten Strahlung der beiden Strahlungsquellen ausgebildet sein. Wenn in dem untersuchten Körpergewebe Sauerstoff verbraucht wird, wird Oxihämoglobin in Desoxihämoglobin umgewandelt. Durch einen Vergleich der aus den unterschiedlichen Volumenbereichen des Körpergewebes stammenden Strahlung der beiden Strahlungsquellen kann die Änderung des Konzentrationsverhältnisses von Oxihämoglobin und Desoxihämoglobin festgestellt werden. Hieraus ergibt sich wiederum der lokale Sauerstoffverbrauch und daraus letztlich der Blutglukosespiegel. Somit ist die Auswertungseinheit der erfindungsgemäßen Messvorrichtung sinnvollerweise eingerichtet zur Bestimmung des lokalen Sauerstoffverbrauchs und/oder des Blutglukosespiegels anhand der Intensitäten der von dem Körpergewebe gestreuten und/oder transmittierten Strahlung der beiden Strahlungsquellen.

Gemäß einer besonders vorteilhaften Ausgestaltung sind sämtliche Komponenten der erfindungsgemäßen Messvorrichtung in einem gemeinsamen Gehäuse angeordnet. Dabei weist die Vorrichtung an einem Ende des Gehäuses einen Messkopf mit den benötigten Messsensoren auf. Auf diese Weise ist die Messvorrichtung handführbar und kann, entweder vom Benutzer selbst oder vom behandelnden Arzt, genutzt werden, um den gesamten Körper systematisch auf krankhafte Veränderungen hin zu untersuchen. In das Gehäuse sollte eine Anzeigeeinheit integriert sein, mittels welcher die lokale Sauerstoffkonzentration des Blutes und/oder die gemäß der Erfindung ermittelten lokalen metabolischen Parameter für den Arzt oder den Benutzer anzeigbar sind.

Weiterhin kann der Messkopf der erfindungsgemäßen Messvorrichtung Elektroden zur bioelektrischen Impedanzmessung und zur EKG-Messung umfassen. Für eine Zweipunktmessung kann eine weitere EKG-Elektrode in das Gehäuse der Messvorrichtung integriert sein. Diese weitere EKG-Elektrode kann gleichzeitig zur bioelektrischen Impedanzmessung, nämlich zur Messung von globalen Gewebeparametern, wie globaler Fettgehalt und/oder globaler Wassergehalt, genutzt werden. Die Anordnung der Elektroden ist zweckmäßigerweise so gewählt, dass eine bioelektrische Impedanzmessung von einem Arm des Benutzers zum anderen Arm möglich ist. Außerdem kann in den Messkopf wenigstens ein Wärmesensor zur Bestimmung der über die Hautoberfläche abgegebenen Wärme integriert sein. Besonders praktisch ist es, wenn bei der erfindungsgemäßen Messvorrichtung die für die verschiedenen Messverfahren (Oximetrie, bioelektrische Impedanzmessung, Wärmemessung, EKG, Messung des Hautkolorits) benötigten Messsensoren in einem einzigen Messkopf zusammengefasst sind. Durch diese Ausgestaltung des Messkopfes ist sichergestellt, dass sämtliche Messwerte gleichzeitig an dem jeweils interessierenden Messort erfasst werden.

Die erfindungsgemäße Messvorrichtung kann miniaturisiert ausgebildet sein und kann in einen am Körper eines Benutzers getragenen Gegenstand, wie beispielsweise eine Armbanduhr, ein Brillengestell oder auch ein Kleidungsstück, integriert sein. Es ist dann eine kontinuierliche Überwachung des Gesundheitszustands möglich.

Die Erfindung betrifft weiterhin ein Verfahren zur Erfassung und Auswertung von physiologischen Parametern gemäß Anspruch 28.

Die der Erfindung zugrunde liegende Aufgabe wird bei einem solchen Verfahren dadurch gelöst, dass mittels der Auswertungseinheit wenigstens ein lokaler metabolischer Parameter, insbesondere der lokale Sauerstoffverbrauch, aus den oximetrischen Signalen bestimmt wird.

Sinnvollerweise werden zusätzlich lokale Gewebeparameter, wie Fettgehalt, Wassergehalt und/oder Durchblutung, erfasst, wobei der wenigstens eine lokale metabolische Parameter aus den oximetrischen Signalen und den lokalen Gewebeparametern bestimmt wird. Die lokalen Gewebeparameter können mittels bioelektrischer Impedanzmessung, optisch oder mittels Wärmemessung erfasst werden. Sinnvoll ist es, zusätzlich die Erfassung eines EKG-Signals durchzuführen. Mittels der Auswertungseinheit kann ein kardiovaskulärer Parameter aus den plethysmographischen Messsignalen und dem EKG-Signal bestimmt werden. Besonders sinnvoll ist es, wie oben ausgeführt, wenn zusätzlich globale Gewebeparameter, wie Fettgehalt und/oder Wassergehalt, erfasst werden. Es kann dann basierend auf dem mittels der Auswertungseinheit gewonnenen kardiovaskulären Parameter und den globalen Gewebeparametern ein globaler Fitnessindex berechnet werden. Bei einer besonders vorteilhaften Variante des erfindungsgemäßen Verfahrens werden mittels der optischen Messeinheit unterschiedliche Volumenbereiche des untersuchten Körpergewebes bestrahlt, wobei - wie oben beschrieben - der wenigstens eine lokale metabolische Parameter aus der von dem Körpergewebe in den unterschiedlichen Volumenbereichen gestreuten und/oder transmittierten Strahlung bestimmt wird. Hierzu kann die optische Messeinheit wenigstens zwei Strahlungsquellen mit unterschiedlichen räumlichen Abstrahlcharakteristiken umfassen, wobei der lokale Sauerstoffverbrauch und/oder der Blutglukosespiegel anhand der Intensitäten der von dem Körpergewebe gestreuten und/oder transmittierten Strahlung der beiden Strahlungsquellen bestimmt werden.

Ein Ausführungsbeispiel der Erfindung wird im Folgenden unter Bezugnahme auf die Zeichnungen näher erläutert. Es zeigen:
- Figur 1: schematische Ansicht einer Messvorrichtung mit vergrößerter Darstellung des Messkopfes;
- Figur 2: Darstellung der Vorrichtung anhand eines Blockdiagramms;
- Figur 3: Blockdiagramm-Darstellung der Oximetrieeinheit der Messvorrichtung;
- Figur 4: Blockdiagramm-Darstellung der Wärmemesseinheit;
- Figur 5: Blockdiagramm-Darstellung der Impedanzmesseinheit der Messvorrichtung;
- Figur 6: Blockdiagramm-Darstellung der EKG-Einheit der Messvorrichtung;
- Figur 7: Darstellung der Signalauswertung mittels der Messvorrichtung;
- Figur 8: schematische Darstellung einer alternativen Anordnung von Strahlungsquellen, Strahlungssensoren und Elektroden zur bioelektrischen Impedanzmessung bei der Messvorrichtung;
- Figur 9: Illustration einer Realisierungsmöglichkeit einer erfindungsgemäßen Messvorrichtung mit zwei Strahlungsquellen.

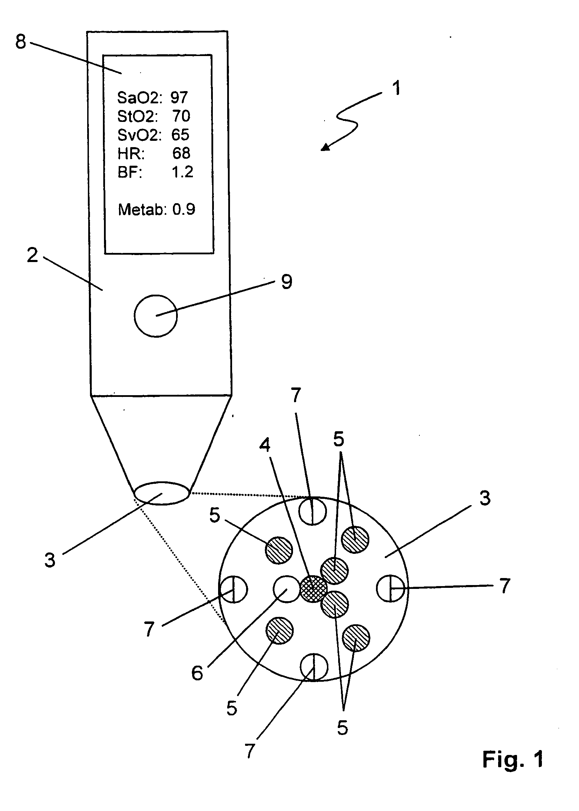

In der Figur 1 ist die Messvorrichtung insgesamt mit der Bezugsziffer 1 bezeichnet. Sämtliche Komponenten der Vorrichtung sind in einem gemeinsamen Gehäuse 2 untergebracht, so dass das Gerät per Hand an beliebigen Messorten am Körper eines Benutzers zum Einsatz gebracht werden kann. Am vorderen Ende des Gehäuses 2 ist ein Messkopf 3 angeordnet, in den die verschiedenen Messsensoren der Vorrichtung 1 integriert sind. Diese werden bei der Verwendung der Messvorrichtung 1 auf die Hautoberfläche des Benutzers am Messort aufgesetzt. Der Messkopf umfasst eine zentral angeordnete Leuchtdiode 4, die dazu in der Lage ist, Licht bei verschiedenen Wellenlängen zu emittieren. Hierzu können beispielsweise verschiedene lichtemittierende Halbleiterelemente in einem gemeinsamen Gehäuse der Leuchtdiode 4 untergebracht sein. Ebenso denkbar ist die Verwendung von Lichtwellenleitern, um das Licht von verschiedenen Lichtquellen an die Unterseite des Messkopfes 3 zu führen. Des Weiteren umfasst der Messkopf 3 insgesamt sechs Photosensoren 5, die in unterschiedlichem Abstand zu der Lichtquelle 4 angeordnet sind. Zwei der Photosensoren 5 sind direkt neben der Lichtquelle 4 angeordnet. Zwei weitere Sensoren 5 befinden sich in einem mittleren Abstand von der Lichtquelle 4, während die zwei verbleibenden Sensoren 5 in maximalem Abstand zur Lichtquelle 4 angeordnet sind. Die unmittelbar neben der Lichtquelle 4 angeordneten Sensoren 5 empfangen hauptsächlich das an den oberen Hautschichten des Benutzers gestreute Licht. Demgegenüber sind die weiter von der Lichtquelle 4 entfernten Sensoren 5 geeignet, die Lichtabsorption in tieferen Gewebeschichten zu messen. Weiterhin ist unmittelbar neben der Lichtquelle 4 ein Wärmesensor 6 vorgesehen. Dadurch ist gewährleistet, dass die Bestimmung der Durchblutung anhand der Wärmemessung am selben Messort erfolgt wie die optische Messung. Außen am Messkopf 3 sind vier Elektroden 7 zur Messung der lokalen bioelektrischen Impedanz vorgesehen. Die Elektroden sind jeweils zweigeteilt und bestehen aus zwei voneinander elektrisch isolierten, separaten Kontaktflächen. Dabei dient jeweils eine der beiden Kontaktflächen zur Aufprägung eines elektrischen Stromes am Messort, während die andere Kontaktfläche zur Spannungsmessung genutzt wird. Auf diese Weise wird sichergestellt, dass die Messergebnisse nicht von den Kontaktwiderständen der Messelektroden beeinflusst sind. Die vier Elektroden 7 können bei der bioelektrischen Impedanzmessung in verschiedenen Kombinationen genutzt werden, um dadurch die Zuverlässigkeit des Messergebnisses zu optimieren. Zumindest eine der Elektroden 7 wird außerdem als EKG-Elektrode einer EKG-Einheit der Messvorrichtung 1 verwendet. In das Gehäuse 2 der Messvorrichtung 1 ist ein LCD-Display 8 als Anzeigeeinheit integriert. Das LCD-Display 8 dient zur Anzeige der lokalen Sauerstoffkonzentration des Blutes. Dabei werden separat die arterielle (SaO₂), die kapillare (StO₂) und die venöse (SvO₂) Sauerstoffsättigung angezeigt. Angezeigt wird weiterhin die ermittelte Herzfrequenz (HR), der lokal bestimmte Fettgehalt des Gewebes (BF). Schließlich wird noch ein lokaler metabolischer Parameter angezeigt, der die Stoffwechselaktivität am Messort wiedergibt. Außerdem ist am Gehäuse 2 ein Ein-/Ausschalter 9 angeordnet, der in üblicher Weise zur Aktivierung bzw. zur Deaktivierung des Gerätes dient. Die Betätigungsfläche des Ein-/Ausschalters 9 bildet außerdem die Kontaktfläche einer weiteren EKG-Elektrode, sodass eine einfache Zweipunktableitung des EKG-Signals des Benutzers der Vorrichtung erfolgen kann. Über die Kontaktfläche ist außerdem eine Arm-zu-Arm-Messung von globalen Gewebeparametern, wie globaler Fettgehalt und/oder globaler Wassergehalt, durch bioelektrische Impedanzmessung möglich.

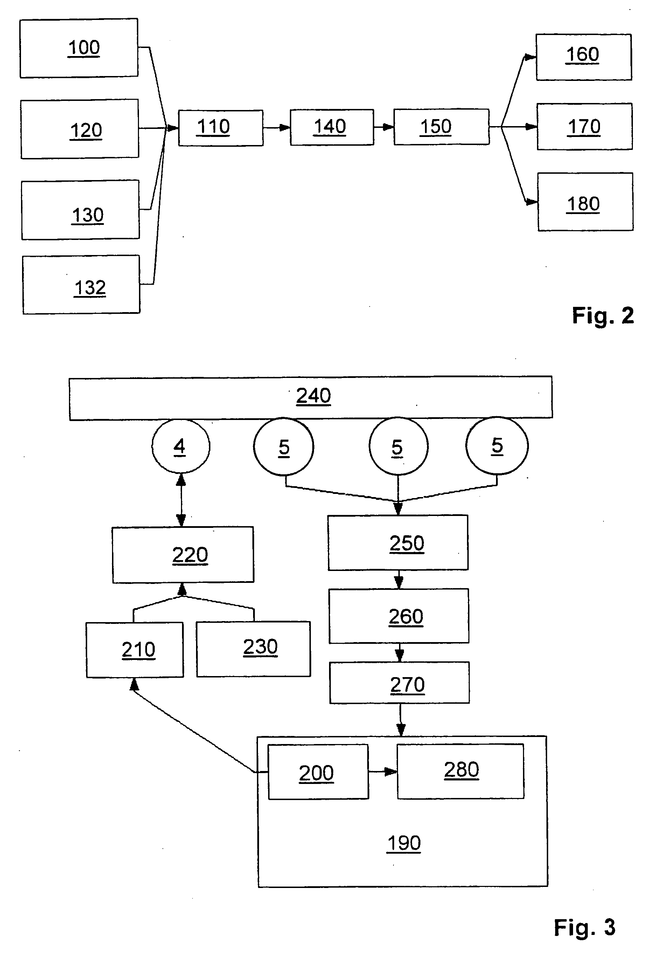

Die Figur 2 zeigt schematisch den Aufbau der Messvorrichtung als Blockdiagramm. Die Vorrichtung 1 umfasst eine optische Messeinheit 100 zur optischen Messung der Sauerstoffkonzentration im Blutgefäßsystem des Körpergewebes am jeweiligen Messort. Außerdem dient die optische Messeinheit 100 der Messung des lokalen Hautkolorits. Die mittels der optischen Messeinheit 100 erfassten oximetrischen und plethysmographische Signale werden einer Analyseeinheit 110 zugeführt. Eine weitere wesentliche Komponente der Vorrichtung 1 ist eine Wärmemesseinheit 120 zur Bestimmung der über die Hautoberfläche abgegebenen Wärme. Dabei arbeitet die Wärmemesseinheit 120 orts-, zeit- und tiefenaufgelöst. Auch die mittels der Wärmemesseinheit 120 erfassten Messsignale werden der Analyseeinheit 110 zur Weiterverarbeitung zugeführt. Außerdem umfasst die Messvorrichtung 1 eine Impedanzmesseinheit 130, die zur Erfassung von lokalen Gewebeparametern mittels bioelektrischer Impedanzmessung dient. Die Messsignale der Impedanzmesseinheit 130 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 100 sind die Lichtquelle 4 sowie die Lichtsensoren 5 des in der Figur 1 dargestellten Messkopfes 3 zugeordnet. Die Wärmemesseinheit 120 ist mit dem Wärmesensor 6 verbunden. Die Impedanzmesseinheit 130 erfasst Messsignale über die Elektroden 7 des Messkopfes 3. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 100, der Wärmemesseinheit 120, der Impedanz-Messeinheit 130 und der EKG-Einheit 132 in eine Auswertungseinheit 140. Die Auswertungseinheit 140 ist dafür zuständig, aus den Messsignalen die für die Diagnose wesentlichen Parameter zu berechnen. Aus den zeitabhängig aufgenommenen Messsignalen der Impedanzmesseinheit 130 wird zunächst die Zusammensetzung des untersuchten Körpergewebes (Wassergehalt, Fettgehalt usw.) berechnet. Aus den Signalen der optischen Messeinheit 100 wird die arterielle Sauerstoffsättigung und - unter Zugrundelegung der zuvor auf der Basis der Impedanzmessung ermittelten Gewebeparameter - die kapillare Sauerstoffsättigung berechnet. Weiterhin werden aus den Messsignalen der Wärmemesseinheit 120 und aus den plethysmographischen Daten, die aus der zeitabhängigen Impedanzmessung ableitbar sind, die Durchblutung und die arterielle Temperatur bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch am Messort berechnet. Weiterhin ist die Auswertungseinheit 140 dafür zuständig, wenigstens einen kardiovaskulären Parameter aus den plethysmographischen Daten und dem EKG-Signal zu bestimmen. Die Berechnungsergebnisse werden mittels einer Diagnoseeinheit 150 interpretiert. Die Diagnoseeinheit 150 dient zur Bewertung der mittels der Auswertungseinheit 140 berechneten lokalen metabolischen und kardiovaskulären Parameter und zur Registrierung von Veränderungen der Parameter in Abhängigkeit vom Messort. Die Diagnoseeinheit 150 ist so eingerichtet, dass diese Hinweise, die auf eine mögliche Erkrankung, wie beispielsweise eine Entzündung, eine Krebserkrankung oder Arteriosklerose, hindeuten, automatisch anhand der Berechnungsergebnisse der Auswertungseinheit 140 erkennt. Einem Benutzer der Messvorrichtung 1 kann dann ein entsprechender Hinweis über die Anzeigeeinheit 8 der Vorrichtung 1 gegeben werden. Ein solcher Hinweis kann beispielsweise in Form eines globalen Indexparameters über die Anzeigeeinheit 8 ausgegeben werden, der leicht und eindeutig interpretierbar ist und eine Aussage über die Wahrscheinlichkeit einer vorliegenden Erkrankung ermöglicht. Die Auswertungseinheit 140 und die Diagnoseeinheit 150 sind zur Anzeige der Messresultate mit einer Grafikeinheit 160 verbunden, die ihrerseits die Anzeigeeinheit 8 der Messvorrichtung 1 ansteuert. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Verbindung der Messvorrichtung 1 mit einem Computer oder einem anderen Kommunikationsgerät dient. Über die Schnittstelleneinheit 180 können sämtliche Daten und Parameter, insbesondere auch die in der Speichereinheit 170 gespeicherten Daten und Parameter, an einen nicht näher dargestellten PC eines behandelnden Arztes übertragen werden. Dort können die Daten detaillierter analysiert werden. Insbesondere können über einen längeren Zeitraum mit der Vorrichtung 1 aufgenommene Daten und Parameter auf Veränderungen hin untersucht werden, um daraus Schlussfolgerungen hinsichtlich der Entwicklung einer bestehenden Erkrankung ableiten zu können. Außerdem besteht die Möglichkeit, die erfindungsgemäße Messvorrichtung 1 als bloße Messdatenerfassungs- und -sendeeinheit zu benutzen, wobei die aufgenommenen Signale direkt an den PC des Arztes übertragen werden. Mittels des PCs können die entsprechenden Auswertungen und Berechnungen dann gegebenenfalls schneller und komfortabler durchgeführt werden.

Die Figur 3 illustriert den Aufbau der optischen Messeinheit 100 der Vorrichtung 1. Die optische Messeinheit 100 umfasst einen Mikrokontroller 190. Bestandteil des Mikrokontrollers 190 ist ein Timing-Generator 200. Dieser erzeugt Steuerungssignale, die einer Modulationseinheit 210 zugeführt werden. Dadurch wird die zeitliche Modulation der Lichtemission der Leuchtdiode 4 gesteuert. Die Leuchtdiode 4 ist über eine Regelungseinheit 220 mit der Modulationseinheit 210 verbunden. Die Intensität des von der Leuchtdiode 4 emittierten Lichtes ist außerdem über eine Leistungssteuerungseinheit 230 anpassbar. Die Leuchtdiode 4 ist dazu in der Lage, Licht bei zumindest drei verschiedenen Wellenlängen zu emittieren. Hierzu sind verschiedene Licht emittierende Halbleiterbauelemente in einem einzigen Gehäuse der Leuchtdiode 4 vereinigt. Mittels des Timing-Generators 200 wird die zeitliche Abfolge der Lichtemission bei den verschiedenen Lichtwellenlängen gesteuert. Die in den Messkopf 3 der Vorrichtung 1 integrierten Photosensoren 5 sind ebenso wie die Leuchtdiode 4 mit dem in der Figur 3 schematisch angedeuteten Körpergewebe 240 des Benutzers in Kontakt. In dem Körpergewebe 240 wird das Licht der Leuchtdiode 4 gestreut und entsprechend der Sauerstoffkonzentration des Blutes, das das Gewebe 240 durchströmt, absorbiert. Das gestreute Licht wird von den Photosensoren 5 registriert. Der Photostrom jedes Photosensors 5 wird mittels eines Konverters 250 in eine Spannung umgewandelt, mittels eines Verstärkers 260 verstärkt und mittels eines Analog/Digital-Wandlers 270 in digitale Messsignale umgewandelt. Die Digitalsignale werden sodann einem Demodulator 280 zugeführt, der Bestandteil des Mikrokontrollers 190 ist. Der Demodulator 280 separiert die aufgenommenen Messsignale nach den entsprechenden Lichtwellenlängen und nach den unterschiedlichen Entfernungen zwischen den Photosensoren 5 und der Leuchtdiode 4. Schließlich werden die Signale an die Analyseeinheit 110 weitergegeben.

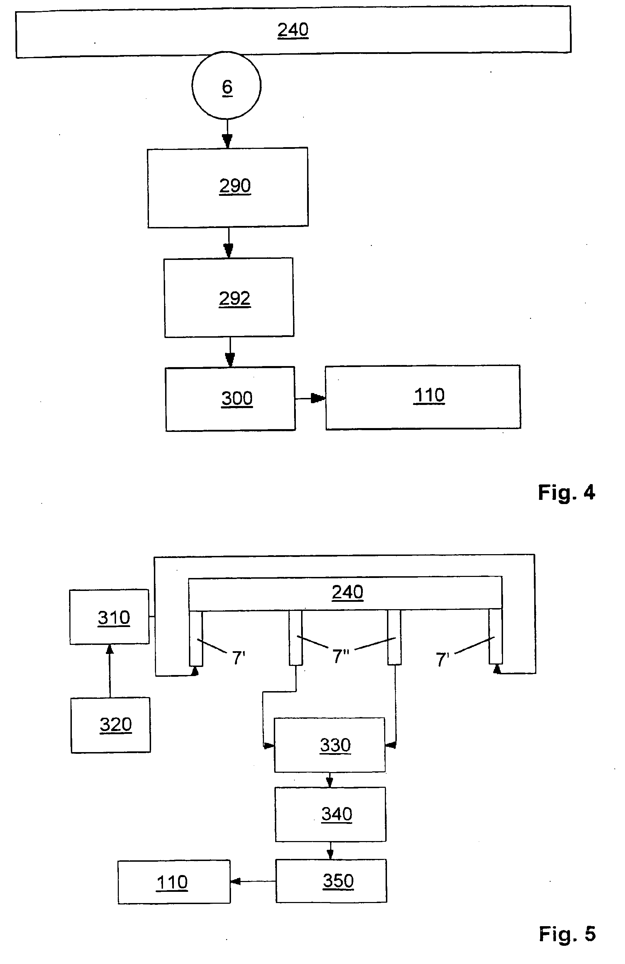

Anhand der Figur 4 wird der Aufbau der Wärmemesseinheit 120 der Messvorrichtung erläutert. Der Wärmesensor 6, der mit dem Körpergewebe 240 in Berührung ist, weist mehrere nicht näher dargestellte Temperaturmesselemente sowie ein wärmeleitendes Element auf. Sobald der Sensor 6 mit dem Gewebe 240 in Kontakt kommt, beginnt ein Wärmeaustausch. Mittels der Temperaturmesselemente wird die Temperatur an verschiedenen Stellen an dem wärmeleitenden Element des Sensors 6 gemessen. Hieraus kann die von dem Gewebe 240 lokal abgegebene Wärme (orts-, zeit- und tiefenaufgelöst) bestimmt werden. Die mittels der Temperaturmesselemente erfassten Signale durchlaufen einen Impedanzwandler 290 sowie einen Verstärker 292 und werden mittels eines Analog/Digital-Wandlers 300 digitalisiert. Die digitalen Messsignale werden sodann der Analyseeinheit 110 zur weiteren Verarbeitung zugeführt. Ein geeigneter Wärmesensor 6 ist beispielsweise in der Veröffentlichung von Ok Kyung Cho et al. (Ok Kyung Cho, Yoon Ok Kim, Hiroshi Mitsumaki, Katsuhiko Kuwa, "Noninvasive Measurement of Glucose by Metabolic Heat Conformation Method", Clinical Chemistry 50, 2004, Nr. 10, S. 1894 bis 1898) beschrieben.

In der Figur 5 ist der Aufbau der Impedanzmesseinheit 130 der Messvorrichtung 1 dargestellt. Die Impedanzmesseinheit 130 umfasst mehrere Elektroden 7. Über Kontaktflächen 7' wird dem untersuchten Körpergewebe 240 ein Wechselstrom aufgeprägt, der mittels einer Stromquelle 310 erzeugt wird. Die Stromquelle 310 wird von einem Sinusgenerator 320 angesteuert. Die Frequenz des Wechselstroms variiert zwischen 20 kHz und 100 kHz. Über Kontaktflächen 7" wird eine Spannung als Messsignal am Körpergewebe 240 abgegriffen. Aus dem Verhältnis der gemessenen Spannung zu dem aufgeprägten Strom kann auf die lokale Impedanz des Körpergewebes 240 zurückgeschlossen werden. Hierzu wird die Spannung mittels eines Verstärkers 330 verstärkt und mittels eines Filters 340 gefiltert, um Störsignale zu eliminieren. Wiederum erfolgt eine Digitalisierung mittels eines Analog/Digital-Wandlers 350. Die digitalisierten Messwerte werden wiederum der Analyseeinheit 110 zur weiteren Verarbeitung zugeführt.

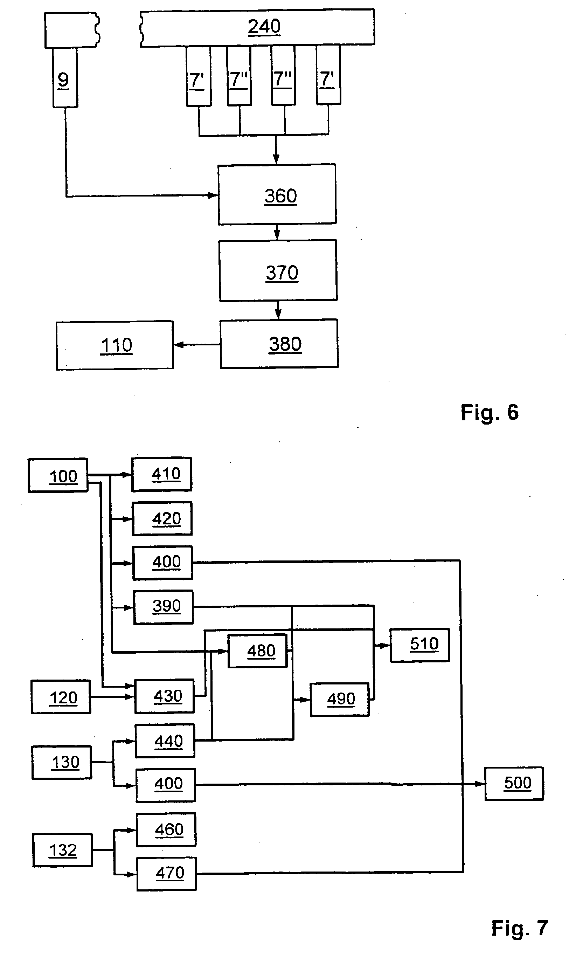

Anhand der Figur 6 wird der Aufbau der EKG-Einheit 132 der Messvorrichtung veranschaulicht. Die EKG-Einheit 132 erfasst ein EKG-Signal über EKG-Elektroden 7' bzw. 7". Es sind dies die Elektroden der Impedanzmesseinheit 130. Die Elektroden 7' und 7" haben also bei dem dargestellten Ausführungsbeispiel eine Doppelfunktion. Für eine brauchbare Zweipunktableitung des EKG-Signals ist eine weitere EKG-Elektrode 9 erforderlich, die in ausreichender räumlicher Entfernung von den Elektroden 7' und 7" mit dem Körper des Benutzers in Kontakt kommt. Die EKG-Elektrode 9 bildet bei dem Ausführungsbeispiel gleichzeitig die Bedienfläche des Ein-/Ausschalters der Messvorrichtung 1. Somit sind sämtliche Elektroden in die Messvorrichtung 1 integriert. Separate, z. B. über Kabel angeschlossene Elektroden sind (für eine einfache Zweipunktableitung des EKG-Signals) nicht zwingend erforderlich. Statt der Bedienfläche des Schalters der Messvorrichtung 1 kann ebenso gut eine zusätzliche Elektrode am Gehäuse 2 der Messvorrichtung 1 angeordnet werden. Das abgeleitete EKG-Signal wird mittels Verstärker 360 und Filter 370 aufbereitet. Nach Passieren eines weiteren Analog/Digital-Wandlers 380 wird das Signal an die Analyseeinheit 110 weitergegeben.

Der Figur 7 ist die Vorgehensweise bei der Ermittlung der physiologischen Parameter zu entnehmen. Von der optischen Messeinheit 100 werden mittels Pulsoximetrie ein arterieller Sauerstoffsättigungswert 390, mittels Photoplethysmographie ein Volumenpulssignal 400, und mittels Reflexions-/Absorptionsmessung ein Hautkoloritwert 410 geliefert. Außerdem liefert die optische Messeinheit 100 einen Differenzwert 420 zwischen systolischem und diastolischem Volumenpuls. Mittels der Wärmemesseinheit 120 wird die Durchblutung 430 ermittelt. Gleichzeitig kann die Durchblutung 430 auch mittels der optischen Messeinheit 100 gewonnen werden. Die Signale der Impedanzmesseinheit 130 ergeben die lokalen Gewebeparameter 440 und ebenfalls den Volumenpuls 400. Die EKG-Einheit 132 liefert Daten 460 über etwaige Arrhythmien und über die Reizleitung am Herzmuskel. Außerdem ist das eigentliche EKG-Signal 470 mit den Zeitpunkten der Herzaktivitäten verfügbar. Auf der Basis der Gewebeparameter 440 und der arteriellen Sauerstoffsättigung 390 wird die kapillare, d. h. die arteriovenöse Sauerstoffsättigung 480 ermittelt. Daraus wird dann wiederum unter Einbeziehung der Gewebeparameter 440 die venöse Sauerstoffsättigung 490 bestimmt. Aus den Herzschlagzeitpunkten 470 und dem zeitlichen Verlauf der Volumenpulssignale 400 lässt sich die Pulswellengeschwindigkeit 500 ermitteln. Aus diesen Daten kann eine Aussage bezüglich des allgemeinen kardiovaskulären Zustands (der Fitness) des Benutzers getroffen werden. Dabei wird ein kardiovaskulärer Index aus den Arrhythmien 460, der Pulswellengeschwindigkeit 500 und der Differenz zwischen systolischem und diastolischem Volumenpuls 420 ermittelt. In die Indexberechnung wird außerdem der lokale Sauerstoffverbrauch 510 einbezogen, der sich aus der Durchblutung 430, der arteriellen Sauerstoffsättigung 390 und der venösen Sauerstoffsättigung 490 ergibt. Einbezogen werden weiterhin der Koloritwert 410 und der Körperfettgehalt 440.

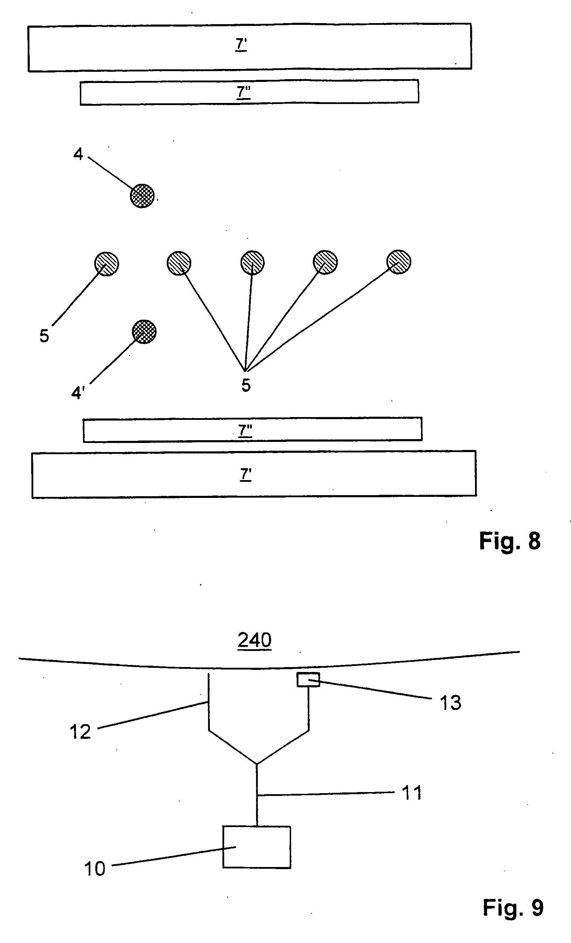

In der Figur 8 ist eine alternative Sensoranordnung der erfindungsgemäßen Messvorrichtung dargestellt. Ähnlich der Darstellung in der Figur 1 zeigt die Figur 8 eine Ansicht der mit der Haut des Benutzers der Vorrichtung in Kontakt zu bringenden Oberfläche des Messkopfes. Die Darstellung in der Figur 8 ist stark vergrößert. Die von den Sensorelementen beanspruchte Fläche kann nur etwa 0,5 bis 2 cm² betragen.

Bei dem in der Figur 8 dargestellten Ausführungsbeispiel sind zwei Strahlungsquellen 4 und 4' vorgesehen, welche unterschiedliche Volumenbereiche des untersuchten Körpergewebes bestrahlen. Hierzu haben die zwei Strahlungsquellen 4 und 4' unterschiedliche räumliche Abstrahlcharakteristiken, nämlich unterschiedliche Abstrahlwinkel. Bei der Strahlungsquelle 4 handelt es sich um eine Leuchtdiode, während es sich bei der Strahlungsquelle 4' um einen Laser, beispielsweise einen so genannten VCSEL-Laser (engl. "vertical cavity surface emitting laser") handelt. Sowohl die Leuchtdiode 4 als auch der Laser 4' emittieren Licht mit sehr ähnlicher Wellenlänge (z. B. 630 nm und 650 nm), aber mit unterschiedlichen Öffnungswinkeln (z. B. 25° und 55°). Mit der in der Figur 8 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 4 und 4' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 8 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobektischer Punkt). Das von der Leuchtdiode bzw. von dem Laser emittierte Licht kann mittels entsprechender Lichtleitfasern an die entsprechende Stelle am Messkopf geführt werden. In diesem Fall sind mit den Bezugsziffern 4 und 4' in der Figur 8 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 4 als auch der Strahlungsquelle 4' wird mittels äquidistant zueinander angeordneten Strahlungssensoren 5 detektiert. Hierbei kann es sich um Fotodioden handeln. Vorzugsweise sind die Fotodioden nicht direkt an der Oberfläche des Messkopfes angeordnet. Stattdessen wird das Licht über Lichtleitfasern den Fotodioden zugeführt. Zur Unterscheidung des Lichtes der Strahlungsquelle 4 von dem Licht der Strahlungsquelle 4' können die beiden Lichtquellen 4 und 4' unterschiedlich zeitlich moduliert betrieben werden, wobei die mittels der Sensoren 5 detektierten Signale entsprechend demoduliert werden. Alternativ ist es möglich, die Strahlung der beiden Strahlungsquellen 4 und 4' anhand der unterschiedlichen Wellenlänge zu unterscheiden. Die Strahlungsintensität der von den Strahlungsquellen 4 und 4' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 8 dargestellten äquidistanten Sensoren 5 können die interessierenden Parameter der Intensitätsschwächung mit großer Genauigkeit bestimmt werden, und zwar getrennt für die von den Strahlungsquellen 4 und 4' jeweils erfassten Volumenbereiche des untersuchten Körpergewebes. Die den verschiedenen Strahlungsquellen 4 und 4' zuzuordnenden Parameter der Intensitätsschwächung können mittels der Auswertungseinheit der erfindungsgemäßen Messvorrichtung zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 4 und 4' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 4 und 4' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits von dem Blutglukosespiegel abhängt, kann mittels der erläuterten differenziellen Messung der Strahlungsabsorption auch der Blutglukosespiegel ermittelt werden. Als sinnvolle Ergänzung wird parallel zur optischen Messung eine Bioimpedanzanalyse durchgeführt, wozu die in der Figur 8 dargestellten Elektroden 7' und 7" vorgesehen sind. Zweck der Bioimpedanzmessung ist vor allem die Bestimmung der lokalen Durchblutung. Diese kann als weiterer Parameter bei der Bestimmung des Sauerstoffverbrauchs und damit auch des Blutglukosespiegels herangezogen werden. Bei dem in der Figur 8 dargestellten Ausführungsbeispiel sind die Elektroden 7' und 7" auf gegenüberliegenden Seiten der Strahlungsquellen 4 und 4' und der Strahlungssensoren 5 angeordnet, um sicherzustellen, dass von der Bioimpedanzmessung und der optischen Messung derselbe Bereich des untersuchten Körpergewebes erfasst ist.

Die Figur 9 zeigt eine Möglichkeit auf, zwei Strahlungsquellen 4 und 4' mit unterschiedlicher räumlicher Abstrahlcharakteristik einfach und kostengünstig zu realisieren. Hierzu wird ein einzelnes Strahlung emittierendes Element 10, beispielsweise eine Leuchtdiode, benutzt, deren Licht in eine Lichtleitfaser 11 eingekoppelt wird. Die Lichtleitfaser ist an einer geeigneten Stelle in zwei Faserzweige aufgespalten. Über den Faserzweig 12 gelangt das Licht direkt in das untersuchte Körpergewebe 240. In dem anderen Faserzweig ist ein zusätzliches optisches Element 13, beispielsweise eine Linse, vorgesehen, beispielsweise um einen kleineren Abstrahlwinkel zu erzielen. Jeder der in der Figur 9 dargestellten Faserzweige bildet somit eine Strahlungsquelle 4 bzw. 4', wie sie in der Figur 8 dargestellt sind.

## Patentansprüche

1. Messvorrichtung zur nicht-invasiven Bestimmung von physiologischen Parametern, mit wenigstens einer optischen Messeinheit (100) zur Erzeugung von oximetrischen und/oder plethysmographischen Messsignalen, und mit einer Auswertungseinheit (140) zur Verarbeitung der Messsignale, wobei die Auswertungseinheit (140) eingerichtet ist zur Bestimmung wenigstens eines lokalen metabolischen Parameters, insbesondere des lokalen Sauerstoffverbrauchs, aus den Signalen der optischen Messeinheit (100), wobei die optische Messeinheit (100) wenigstens zwei Strahlungsquellen (4, 4') zur Bestrahlung des untersuchten Körpergewebes (240) und wenigstens einen Strahlungssensor (5) zur Detektion der von dem Körpergewebe (240) gestreuten und/oder transmittierten Strahlung aufweist,
**dadurch gekennzeichnet,**
**dass** die wenigstens zwei Strahlungsquellen (4, 4') unterschiedliche räumliche Abstrahlcharakteristiken, nämlich unterschiedliche Öffnungswinkel haben und auf diese Weise unterschiedliche Volumenbereiche des untersuchten Körpergewebes (240) bestrahlen.

2. Messvorrichtung nach Anspruch 1, **gekennzeichnet durch** eine Einheit (120, 130) zur Erfassung von lokalen Gewebeparametern, wie Fettgehalt, Wassergehalt und/oder Durchblutung, wobei die Auswertungseinheit (140) eingerichtet ist zur Bestimmung des wenigstens einen lokalen metabolischen Parameters aus den Signalen der optischen Messeinheit (100) und den Gewebeparametern.

3. Messvorrichtung nach Anspruch 2, **dadurch gekennzeichnet, dass** die Einheit zur Erfassung von lokalen Gewebeparametern eine bioelektrische Impedanzmesseinheit (130) umfasst.

4. Messvorrichtung nach Anspruch 3, **dadurch gekennzeichnet, dass** die bioelektrische Impedanzmesseinheit (130) außerdem zur Erfassung von globalen Gewebeparametern, wie globaler Fettgehalt und/oder globaler Wassergehalt, ausgebildet ist.

5. Messvorrichtung nach einem der Ansprüche 2 bis 4, **gekennzeichnet durch** einen Wärmesensor (6) zur Bestimmung der über die Hautoberfläche abgegeben Wärme, wobei die Auswertungseinheit (140) zur Bestimmung der lokalen metabolischen Parameter unter Berücksichtigung der Signale des Wärmesensors (6) eingerichtet ist.

6. Messvorrichtung nach einem der Ansprüche 2 bis 6, **gekennzeichnet, durch** einen optischen Sensor zur ortsaufgelösten Bestimmung des Hautkolorits.

7. Messvorrichtung nach einem der Ansprüche 2 bis 6, **gekennzeichnet durch** eine EKG-Einheit (132) zur Erfassung eines EKG-Signals über zwei oder mehr EKG-Elektroden (7, 9), wobei die Auswertungseinheit (140) zur Auswertung des zeitlichen Verlaufs des EKG-Signals eingerichtet ist.

8. Messvorrichtung nach Anspruch 7, **dadurch gekennzeichnet, dass** die Auswertungseinheit (4) eingerichtet ist zur Auswertung des zeitlichen Verlaufs eines mittels der Vorrichtung erfassten plethysmographischen Signals und/oder zur Bestimmung der Pulswellengeschwindigkeit aus dem zeitlichen Verlauf des EKG-Signals und des plethysmographischen Signals.

9. Messvorrichtung nach einem der Ansprüche 2 bis 8, **gekennzeichnet durch** eine Speichereinheit (170) zur Speicherung der mittels der Auswertungseinheit (140) ermittelten Parameter.

10. Messvorrichtung nach einem der Ansprüche 2 bis 9, **gekennzeichnet durch** eine Diagnoseeinheit (150) zur Bewertung der mittels der Auswertungseinheit (140) ermittelten Parameter und zur Registrierung von Veränderungen der Parameter in Abhängigkeit vom Messort und/oder von der Messzeit.

11. Messvorrichtung nach einem der Ansprüche 1 bis 10, **dadurch gekennzeichnet, dass** die Diagnoseeinheit (150) eingerichtet ist zur Bestimmung des Status des Herz-Kreislauf-Systems aus den mittels der Auswertungseinheit (140) ermittelten Parametern.

12. Messvorrichtung nach den Ansprüchen 4 und 11, **dadurch gekennzeichnet, dass** die Diagnoseeinheit (150) zur Berechung eines globalen Fitnessindex auf der Basis des Status des Herz-Kreislauf-Systems und den globalen Gewebeparametern eingerichtet ist.

13. Messvorrichtung nach einem der Ansprüche 1 bis 12, **dadurch gekennzeichnet, dass** die Auswertungseinheit (140) eingerichtet ist zur Bestimmung des wenigstens einen lokalen metabolischen Parameters aus der von dem Körpergewebe (240) gestreuten und/oder transmittierten Strahlung der beiden Strahlungsquellen (4, 4').

14. Messvorrichtung nach Anspruch 13, **dadurch gekennzeichnet, dass** die Auswertungseinheit (140) weiterhin eingerichtet ist zur Bestimmung des lokalen Sauerstoffverbrauchs und/oder des Blutglukosespiegels anhand der Intensitäten der von dem Körpergewebe (240) gestreuten und/oder transmittierten Strahlung der beiden Strahlungsquellen (4, 4').

15. Messvorrichtung nach einem der Ansprüche 1 bis 14, **dadurch gekennzeichnet, dass** die Wellenlänge der von den beiden Strahlungsquellen jeweils emittierten Strahlung im Bereich zwischen 600 und 700 nm, vorzugsweise zwischen 630 und 650 nm, liegt.

16. Messvorrichtung nach einem der Ansprüche 1 bis 15, **dadurch gekennzeichnet, dass** die Strahlungsquellen (4, 4') und die Strahlungssensoren (5) an einem Messkopf (3) angeordnet sind.

17. Messvorrichtung nach Anspruch 16, **dadurch gekennzeichnet, dass** der Messkopf (3) Elektroden (7) zur bioelektrischen Impedanzmessung umfasst.

18. Messvorrichtung nach Anspruch 17, **dadurch gekennzeichnet, dass** wenigstens eine der Elektroden (7) gleichzeitig als EKG-Elektrode ausgebildet ist.

19. Messvorrichtung nach einem der Ansprüche 16 bis 18, **dadurch gekennzeichnet, dass** der Messkopf (3) wenigstens einen Wärmesensor (6) umfasst.

20. Messvorrichtung nach einem der Ansprüche 16 bis 19, **dadurch gekennzeichnet, dass** der Messkopf (3) am vorderen Ende eines gemeinsamen Gehäuses (2) angeordnet ist, welches die optische Messeinheit (100) und die Auswertungseinheit (140) aufnimmt, sodass das gesamte Gerät (1) handführbar ausgebildet ist.

21. Messvorrichtung nach den Ansprüchen 18 und 20, **dadurch gekennzeichnet, dass** das Gehäuse (2) an der Außenseite eine weitere EKG-Elektrode (9) aufweiset.

22. Messvorrichtung nach Anspruch 21, **dadurch gekennzeichnet, dass** die weitere EKG-Elektrode (9) gleichzeitig zur bioelektrischen Impedanzmessung ausgebildet ist.

23. Messvorrichtung nach einem der Ansprüche 1 bis 22, **gekennzeichnet durch** eine Anzeigeeinheit (8) zur Anzeige der lokalen Sauerstoffkonzentration des Blutes und/oder des wenigstens einen lokalen metabolischen Parameters.

24. Messvorrichtung nach einem der Ansprüche 1 bis 23, **gekennzeichnet durch** eine Schnittstelle (180) zur Verbindung der Messvorrichtung (1) mit einem Computer oder einem anderen Gerät.

25. Messvorrichtung nach einem der Ansprüche 1 bis 24, **dadurch gekennzeichnet, dass** sie miniaturisiert ausgebildet ist und in einen am Körper eines Benutzers getragenen Gegenstand integriert ist.

26. Verfahren zur Erfassung und Auswertung von physiologischen Parametern, unter Verwendung einer Messvorrichtung nach einem der Ansprüche 1 bis 25, wobei
- mittels der optischen Messeinheit (100) oximetrische und/oder plethysmographische Messsignale von Körpergewebe (240) erfasst werden,
- und mittels der Auswertungseinheit (140) die oximetrischen und/oder plethysmographischen Messsignale verarbeitet werden, und zwar zur Bestimmung des Pulses und/oder der lokalen Sauerstoffkonzentration,
**dadurch gekennzeichnet,**
**dass** mittels der Auswertungseinheit (140) wenigstens ein lokaler metabolischer Parameter, insbesondere der lokale Sauerstoffverbrauch, aus den oximetrischen Signalen bestimmt wird.

27. Verfahren nach Anspruch 26, **dadurch gekennzeichnet, dass** lokale Gewebeparameter, wie Fettgehalt, Wassergehalt und/oder Durchblutung, erfasst werden, wobei der wenigstens eine lokale metabolische Parameter aus den oximetrischen Signalen und den lokalen Gewebeparametern bestimmt wird.

28. Verfahren nach Anspruch 27, **dadurch gekennzeichnet, dass** die lokalen Gewebeparameter mittels bioelektrischer Impedanzmessung erfasst werden.

29. Verfahren nach Anspruch 27, **dadurch gekennzeichnet, dass** die lokalen Gewebeparameter optisch erfasst werden.

30. Verfahren nach einem der Ansprüche 26 bis 29, **dadurch gekennzeichnet, dass** die lokalen Gewebeparameter mittels ortsaufgelöster Wärmemessung erfasst werden.

31. Verfahren nach einem der Ansprüche 26 bis 30, **gekennzeichnet durch** eine zusätzlich zur Erfassung der oximetrischen Messsignale durchgeführte Erfassung eines EKG-Signals.

32. Verfahren nach Anspruch 31, **dadurch gekennzeichnet, dass** mittels der Auswertungseinheit (140) ein kardiovaskulärer Parameter aus den piethysmographischen Messsignalen und dem EKG-Signal bestimmt wird.

33. Verfahren nach einem der Ansprüche 26 bis 32, **gekennzeichnet durch** eine Erfassung von globalen Gewebeparametern, wie Fettgehalt und/oder Wassergehalt.

34. Verfahren nach den Ansprüchen 32 und 33, **dadurch gekennzeichnet, dass** basierend auf dem kardiovaskulären Parameter und den globalen Gewebeparametern ein globaler Fitnessindex berechnet wird.

35. Verfahren nach einem der Ansprüche 26 bis 34, **dadurch gekennzeichnet, dass** der wenigstens eine lokale metabolische Parameter aus der von dem Körpergewebe (240) in den unterschiedlichen Volumenbereichen gestreuten und/oder transmittierten Strahlung bestimmt wird.

## Claims

1. A measuring device for non-invasive determination of physiological parameters comprising at least one optical measuring unit (100) for generating oximetric and/or plethysmographic measurement signals, and an evaluation unit (140) for processing the measurement signals, wherein the evaluation unit (140) is adapted to determine at least one local metabolic parameter, in particular local oxygen consumption, from the signals of the optical measuring unit (100), wherein the optical measuring unit (100) has at least two radiation sources (4, 4') for irradiating the investigated body tissue (240) and at least one radiation sensor (5) for detection of the radiation transmitted and/or scattered by the body tissue (240),
**characterised in that**
the at least two radiation sources (4, 4') have different spatial radiation characteristics, namely different spread angles, and **in that** way irradiate different volume regions of the body tissue (240) being investigated.

2. A measuring device according to claim 1 **characterised by** a unit (120, 130) for detecting local tissue parameters such as fat content, water content and/or circulation, wherein the evaluation unit (140) is adapted to determine the at least one local metabolic parameter from the signals of the optical measuring unit (100) and the tissue parameters.

3. A measuring device according to claim 2 **characterised in that** the unit for detecting local tissue parameters includes a bioelectrical impedance measuring unit (130).

4. A measuring device according to claim 3 **characterised in that** the bioelectrical impedance measuring unit (130) is additionally adapted to detect global tissue parameters such as global fat content and/or global water content.

5. A measuring device according to one of claims 2 to 4 **characterised by** a heat sensor (6) for determining the heat given off by way of the surface of the skin, wherein the evaluation unit (140) is adapted to determine the local metabolic parameters having regard to the signals of the heat sensor (6).

6. A measuring device according to one of claims 2 to 6 **characterised by** an optical sensor for positionally resolved determination of the skin colour.

7. A measuring device according to one of claims 2 to 6 **characterised by** an ECG unit (132 for detecting an ECG signal by way of two or more ECG electrodes (7, 9), wherein the evaluation unit (140) is adapted to assess the variation in time of the ECG signal.

8. A measuring device according to claim 7 **characterised in that** the evaluation unit (4) is adapted to evaluate the variation in time of a plethysmographic signal detected by means of the device and/or for determining the pulse wave speed from the variation in time of the ECG signal and the plethysmographic signal.

9. A measuring device according to one of claims 2 to 8 **characterised by** a storage unit (170) for storage of the parameters ascertained by means of the evaluation unit (140).

10. A measuring device according to one of claims 2 to 9 **characterised by** a diagnostic unit (150) for assessment of the parameters ascertained by means of the evaluation unit (140) and for recording changes in the parameters in dependence on the measurement location and/or the measurement time.

11. A measuring device according to one of claims 1 to 10 **characterised in that** the diagnostic unit (150) is adapted to determine the status of the cardiovascular system from the parameters ascertained by means of the evaluation unit (140).

12. A measuring device according to claims 4 and 11 **characterised in that** the diagnostic unit (150) is adapted to calculate a global fitness index on the basis of the status of the cardiovascular system and the global tissue parameters.

13. A measuring device according to one of claims 1 to 12 **characterised in that** the evaluation device (140) is adapted to determine the at least one local metabolic parameter from the radiation of the two radiation sources (4, 4'), that is transmitted and/or scattered by the body tissue (240).

14. A measuring device according to claim 13 **characterised in that** the evaluation unit (140) is further adapted to determine the local oxygen consumption and/or the blood glucose level on the basis of the intensities of the radiation of the two radiation sources (4, 4'), that is transmitted and/or scattered by the body tissue (240).

15. A measuring device according to one of claims 1 to 14 **characterised in that** the wavelength of the radiation respectively emitted by the two radiation sources is in the range of between 600 and 700 nm, preferably between 630 and 650 nm.

16. A measuring device according to one of claims 1 to 15 **characterised in that** the radiation sources (4, 4') and the radiation sensors (5) are arranged on a measuring head (3).

17. A measuring device according to claim 16 **characterised in that** the measuring head (3) includes electrodes (7) for bioelectrical impedance measurement.

18. A measuring device according to claim 17 **characterised in that** at least one of the electrodes (7) is at the same time in the form of an ECG electrode.

19. A measuring device according to one of claims 16 to 18 **characterised in that** the measuring head (3) includes at least one heat sensor (6).

20. A measuring device according to one of claims 16 to 19 **characterised in that** the measuring head (3) is arranged at the front end of a common housing (2) which accommodates the optical measuring unit (100) and the evaluation unit (140) so that the entire arrangement (1) is adapted to be hand-guided.

21. A measuring device according to claims 18 to 20 **characterised in that** the housing (2) has a further ECG electrode (9) at the outside.

22. A measuring device according to claim 21 **characterised in that** the further ECG electrode (9) is at the same time adapted for bioelectrical impedance measurement.

23. A measuring device according to one of claims 1 to 22 **characterised by** a display unit (8) for displaying the local oxygen concentration of the blood and/or the at least one local metabolic parameter.

24. A measuring device according to one of claims 1 to 23 **characterised by** an interface (180) for connecting the measuring device (1) to a computer or another device.

25. A measuring device according to one of claims 1 to 24 **characterised in that** it is of a miniaturised design and is integrated in an article worn on the body of a user.

26. A method of detecting and evaluating physiological parameters using a measuring device according to one of claims 1 to 25, wherein
- oximetric and/or plethysmographic measurement signals of body tissue (240) are detected by means of the optical measuring unit (100), and
- the oximetric and/or plethysmographic measurement signals are processed by means of the evaluation unit (140), more specifically for determining the pulse and/or the local oxygen concentration,
**characterised in that**
at least one local metabolic parameter, in particular the local oxygen consumption, is determined from the oximetric signals by means of the evaluation unit (140).

27. A method according to claim 26 **characterised in that** local tissue parameters such as fat content, water content and/or circulation are detected, wherein the at least one local metabolic parameter is determined from the oximetric signals and the local tissue parameters.

28. A method according to claim 27 **characterised in that** the local tissue parameters are detected by means of bioelectrical impedance measurement.

29. A method according to claim 27 **characterised in that** the local tissue parameters are detected optically.

30. A method according to one of claims 26 to 29 **characterised in that** the local tissue parameters are detected by means of positionally resolved heat measurement.

31. A method according to one of claims 26 to 30 **characterised by** detection of an ECG signal which is implemented in addition to detection of the oximetric measurement signals.

32. A method according to claim 31 **characterised in that** a cardiovascular parameter is determined from the plethysmographic measurement signals and the ECG signal by means of the evaluation unit (140).

33. A method according to one of claims 26 to 32 **characterised by** detection of global tissue parameters such as fat content and/or water content.

34. A method according to claims 32 and 33 **characterised in that** a global fitness index is calculated based on the cardiovascular parameter and the global tissue parameters.

35. A method according to one of claims 26 to 34 **characterised in that** the at least one local metabolic parameter is determined from the radiation transmitted and/or scattered by the body tissue (240) in the different volume regions.

## Revendications

1. Dispositif de mesure pour la détermination non invasive de paramètres physiologiques, comportant au moins une unité de mesure (100) optique pour délivrer des signaux de mesure oxymétriques et/ou pléthysmographiques, et comportant une unité d'analyse (140) pour le traitement des signaux de mesure, ladite unité d'analyse (140) étant configurée pour déterminer au moins un paramètre métabolique local, en particulier la consommation d'oxygène locale, à partir des signaux délivrés par l'unité de mesure (100) optique, ladite unité de mesure (100) optique comportant au moins deux sources de rayonnement (4, 4') pour irradier le tissu corporel (240) examiné et au moins un capteur de rayonnement (5) pour détecter le rayonnement dispersé et/ou transmis par le tissu corporel (240),
**caractérisé en ce que** lesdites au moins deux sources de rayonnement (4, 4') possèdent des caractéristiques de rayonnement dans l'espace différentes, à savoir différents angles d'ouverture, et, de cette manière, irradient différentes zones volumique du tissu corporel (240) examiné.

2. Dispositif de mesure selon la revendication 1, **caractérisé par** une unité (120, 130) pour détecter les paramètres tissulaires locaux, tels que la teneur en graisse, la teneur en eau et/ou l'irrigation sanguine, l'unité d'analyse (140) étant configurée pour déterminer ledit au moins un paramètre métabolique local à partir des signaux de l'unité de mesure (100) optique et des paramètres tissulaires.

3. Dispositif de mesure selon la revendication 2, **caractérisé en ce que** l'unité pour détecter les paramètres tissulaires locaux comprend une unité de mesure de l'impédance bioélectrique (130).

4. Dispositif de mesure selon la revendication, **caractérisé en ce que** l'unité de mesure de l'impédance bioélectrique (130) est configurée, en outre, pour détecter des paramètres tissulaires globaux, tels que la teneur globale en graisse et/ou la teneur globale en eau.

5. Dispositif de mesure selon l'une quelconque des revendications 2 à 4, **caractérisé par** un capteur de chaleur (6) pour déterminer la chaleur dégagée à travers la surface de la peau, l'unité d'analyse (140) étant configurée pour déterminer les paramètres métaboliques locaux en tenant compte des signaux du capteur de chaleur (6).

6. Dispositif de mesure selon l'une quelconque des revendications 2 à 5, **caractérisé par** un capteur optique pour déterminer par résolution locale la coloration de la peau.

7. Dispositif de mesure selon l'une quelconque des revendications 2 à 6, **caractérisé par** un électrocardiographe (132) pour détecter un signal ECG par l'intermédiaire de deux électrodes (7, 9) ou plus, l'unité d'analyse (140) étant configurée pour analyser la courbe en fonction du temps du signal ECG.

8. Dispositif de mesure selon la revendication 7, **caractérisé en ce que** l'unité d'analyse (140) est configurée pour analyser la courbe en fonction du temps d'un signal pléthysmographique, détecté par ledit dispositif, et/ou pour déterminer la vitesse de l'onde de pouls à partir de la courbe en fonction du temps du signal ECG et du signal pléthysmographique.

9. Dispositif de mesure selon l'une quelconque des revendications 2 à 8, **caractérisé par** une unité de mémoire (170) pour stocker les paramètres déterminés au moyen de l'unité d'analyse (140).

10. Dispositif de mesure selon l'une quelconque des revendications 2 à 9, **caractérisé par** une unité de diagnostic (150) pour évaluer les paramètres déterminés au moyen de l'unité d'analyse (140) et pour enregistrer les modifications des paramètres en fonction du lieu de mesure et/ou de l'instant de mesure.

11. Dispositif de mesure selon l'une quelconque des revendications 1 à 10, **caractérisé en ce que** l'unité de diagnostic (150) est configurée pour déterminer l'état du système cardio-circulatoire à partir des paramètres déterminés au moyen de l'unité d'analyse (140).

12. Dispositif de mesure selon les revendications 4 et 11, **caractérisé en ce que** l'unité de diagnostic (150) est configurée pour calculer un indice de forme physique global sur la base de l'état du système cardio-circulatoire et les paramètres tissulaires globaux.

13. Dispositif de mesure selon l'une quelconque des revendications 1 à 12, **caractérisé en ce que** l'unité d'analyse (140) est configurée pour déterminer ledit au moins un paramètre métabolique local à partir du rayonnement des deux sources de rayonnement (4, 4'), dispersé et/ou transmis par le tissu corporel (240).

14. Dispositif de mesure selon la revendication 13, **caractérisé en ce que** l'unité d'analyse (140) est configurée, en outre, pour déterminer la consommation d'oxygène locale et/ou la glycémie à l'appui des intensités du rayonnement des deux sources de rayonnement (4, 4'), dispersé et/ou transmis par le tissu corporel (240).

15. Dispositif de mesure selon l'une quelconque des revendications 1 à 14, **caractérisé en ce que** les longueurs d'onde du rayonnement, émis respectivement par les deux sources de rayonnement, se situent dans la plage entre 600 et 700 nm, de préférence entre 630 et 650 nm.

16. Dispositif de mesure selon l'une quelconque des revendications 1 à 15, **caractérisé en ce que** les sources de rayonnement (4, 4') et les capteurs de rayonnement (5) sont agencés sur une tête de mesure (3).

17. Dispositif de mesure selon la revendication 16, **caractérisé en ce que** la tête de mesure (3) comprend des électrodes (7) pour la mesure de l'impédance bioélectrique.

18. Dispositif de mesure selon la revendication 17, **caractérisé en ce qu'**au moins une des électrodes (7) est réalisée en même temps comme électrode d'électrocardiographe.

19. Dispositif de mesure selon l'une quelconque des revendications 16 à 18, **caractérisé en ce que** la tête de mesure (3) comprend au moins un capteur de chaleur (6).

20. Dispositif de mesure selon l'une quelconque des revendications 16 à 19, **caractérisé en ce que** la tête de mesure (3) est agencée au niveau de l'extrémité avant d'un boîtier (2) commun, qui reçoit l'unité de mesure (100) optique et l'unité d'analyse (140), de telle sorte que l'ensemble de l'appareil (1) peut être manipulé manuellement.

21. Dispositif de mesure selon les revendications 18 et 20, **caractérisé en ce que** le boîtier (2) comporte une électrode d'électrocardiographe (9) supplémentaire sur sa face extérieure.

22. Dispositif de mesure selon la revendication 21, **caractérisé en ce que** l'électrode d'électrocardiographe (9) supplémentaire est réalisée en même temps pour la mesure de l'impédance bioélectrique.

23. Dispositif de mesure selon l'une quelconque des revendications 1 à 22, **caractérisé par** une unité d'affichage (8) pour afficher la concentration locale en oxygène du sang et/ou ledit au moins un paramètre métabolique local.

24. Dispositif de mesure selon l'une quelconque des revendications 1 à 23, **caractérisé par** une interface (180) permettant de relier le dispositif de mesure (1) à un ordinateur ou à un autre appareil.

25. Dispositif de mesure selon l'une quelconque des revendications 1 à 24, **caractérisé en ce qu'**il est miniaturisé et est intégré dans un objet qu'un utilisateur porte sur le corps.

26. Procédé permettant de détecter et d'analyser des paramètres physiologiques moyennant l'utilisation d'un dispositif de mesure selon l'une quelconque des revendications 1 à 25,
- les signaux de mesure oxymétriques et/ou pléthysmographiques du tissu corporel (240) étant détectés au moyen de l'unité de mesure (100) optique,
- et les signaux de mesure oxymétriques et/ou pléthysmographiques étant traités au moyen de l'unité d'analyse (140), à savoir pour déterminer le pouls et/ou la concentration locale en oxygène,
**caractérisé en ce qu'**au moins un paramètre métabolique local, en particulier la consommation d'oxygène locale, est déterminé au moyen de l'unité d'analyse (140) à partir des signaux oxymétriques.

27. Procédé selon la revendication 26, **caractérisé en ce que** les paramètres tissulaires locaux, tels que la teneur en graisse, la teneur en eau et/ou l'irrigation sanguine, sont déterminés, ledit au moins un paramètre métabolique local étant déterminé à partir des signaux oxymétriques et des paramètres tissulaires locaux.

28. Procédé selon la revendication 27, **caractérisé en ce que** les paramètres tissulaires locaux sont détectés au moyen de la mesure de l'impédance bioélectrique.

29. Procédé selon la revendication 27, **caractérisé en ce que** les paramètres tissulaires locaux sont détectés par voie optique.

30. Procédé selon l'une quelconque des revendications 26 à 29, **caractérisé en ce que** les paramètres tissulaires locaux sont détectés au moyen d'une mesure de la chaleur à résolution locale.

31. Procédé selon l'une quelconque des revendications 26 à 30, **caractérisé par** une détection d'un signal ECG, effectuée en plus de la détection des signaux de mesure oxymétriques.

32. Procédé selon la revendication 31, **caractérisé en ce qu'**un paramètre cardiovasculaire est déterminé au moyen de l'unité d'analyse (140) à partir des signaux de mesure pléthysmographiques et du signal ECG.

33. Procédé selon l'une quelconque des revendications 26 à 32, **caractérisé par** une détection de paramètres tissulaires globaux, tels que la teneur en graisse et/ou la teneur en eau.

34. Procédé selon les revendications 32 et 33, **caractérisé en ce qu'**un indice de forme physique global est calculé sur la base du paramètre cardiovasculaire et des paramètres tissulaires globaux.

35. Procédé selon l'une quelconque des revendications 26 à 34, **caractérisé en ce que** ledit au moins un paramètre métabolique local est déterminé à partir du rayonnement dispersé et/ou transmis par le tissu corporel (240) dans les différentes zones volumiques.
